# EUROPEAN PATENT SPECIFICATION

(11) **EP 2 346 572 B1**
(45) Date of publication and mention of the grant of the patent: **28.06.2017**
(21) Application number: 09741230.8
(22) Date of filing: 20.10.2009
(51) Int. Cl.: A61Q 5/04, A61K 8/46, A61K 8/81, A61K 8/44

(54) **COMPOSITION FOR THE PERMANENT SHAPING OF HUMAN HAIR**
ZUSAMMENSETZUNG ZUR DAUERHAFTEN VERFORMUNG DES MENSCHLICHEN HAARES
COMPOSITION DE MISE EN FORME PERMANENTE DE CHEVEUX HUMAINS

(30) Priority: 20.10.2008 EP 08018289
(43) Date of publication of application: 27.07.2011
(73) Proprietor: Kao Germany GmbH, 64297 Darmstadt (DE)
(72) Inventor: WOOD, Jonathan, 69469 Weinheim (DE); HULLMANN, Alexandra, 64331 Weinheim (DE); GRIT, Mustafa, 64579 Gernsheim (DE)
(74) Representative: Grit, Mustafa
(86) International application number: PCT/EP2009/007503
(87) International publication number: WO 2010/046081

(56) References cited:
- EP-A- 1 797 861
- EP-A- 1 800 657
- WO-A-94/06410
- DE-A1- 10 360 688
- GB-A- 2 170 830
- US-A1- 2004 120 914
- US-A1- 2007 190 008

## Description

The present invention concerns a composition for the permanent shaping of human hair used both for the permanent waving of human hair with an excellent waving effect as well as for the straightening of either naturally or chemically curled hair.

It is generally known that permanent waving is carried out in two steps, the reductive splitting of the cysteine disulfide bonds in the hair by a reducing agent and the subsequent neutralization by application of an oxidizing agent, whereby the cysteine disulfide bonds are restored.

The reducing agent still most frequently used today is thioglycolic acid, also in form of the salts thereof, in particular its ammonium salt, although numerous other thio compounds have been proposed for this purpose, which, however, mostly did not succeed.

The compositions containing thioglycolates are customarily applied at a pH-value between 7 and 10, in particular 8.5 and 9.5.

Such compositions vary in their waving and/or straightening performance and, therefore, there is still need for further improvement, especially in permanent shaping of damaged to strongly damaged hair and in particular for permanent shaping hair streaks including parts with various damage level in length.

Use of polymers in permanent shaping compositions for hair has been known for many years. For examples in EP 797861 A1 permanent shaping compositions are disclosed comprising homopolymer of dimethyl diallyl ammonium chloride. In an earlier application which has not been published yet for our company, permanent shaping compositions have been disclosed comprising copolymer of vinylpyrrolidone and quaternized vinylimidazole.

The present invention starts from the task of providing a composition for the permanent shaping of human hair with excellent waving and straightening performance. Hair waved or straightened with composition disclosed herein looks and feels natural upon touching by hand. For waved hair it is especially important that the hair has excellent elasticity and bounce.

Accordingly, the first object of the present invention is a composition for permanent shaping and/or straightening hair based on at least one reducing agent further comprising a cationic polymer consisting of a copolymer of vinylpyrrolidone and vinylimidazole and a homopolymer of dimethyl dially ammonium chloride. Vinylimidazole in the copolymer is not quaternised. Cationic charge density of the polymer is preferably between 3 and 4 mEq/g and particularly 3.7 mEq/g at pH 7.0. Recently a cationic polymer has become commercially available under the trade name Luviquat Sensation from BASF with CTFA name Polyquaternium-87 which is the polymer used in the compositions of the present invention. Composition of the present invention comprises cationic polymer at a concentration of 0.1 to 2.5%, preferably 0.2 to 2%, more preferably 0.25 to 1.5% and most preferably 0.25 to 1% by weight calculated to total composition.

The permanent shaping compositions according to the invention comprise at least one reducing compound at a concentration of at least 0.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1.2-propyleneglycol monothioglycolate (see also WO-A 93/1791), 1-3-propanediol monothioglycolate or the isomer mixture resulting therefrom, 1.3-butanediol and 1.4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 0.5 to 15 %, preferably 1.0 to 12.5%, more preferably 2.0 to 12.5% by weight, calculated to total composition as free thioglycolic acid as reference substance.

The permanent shaping compositions containing reducing agents can, if necessary, comprise alkalizing agents. Their quantity is dependent on the reducing agent and the desired pH-value of the composition. Reducing agent compositions preferably contain 0.1% to 5%, in particular 0.5% to 2.5% by weight thereof, calculated to the total composition. Alkalizing agents preferred within the scope of the invention are ammonium carbamate, ammonia and/or ammonium(bi)carbonate, monoethanolamine and triethanolamine. It is desirable to adjust the pH-value between about 6.5 and 10.5, preferably about 7 to 9.5.

Permanent shaping compositions according to the invention are suited for use both for the permanent waving, i.e. curling of human hair and for the straightening, i.e. smoothing thereof.

The viscosity best suited for the permanent shaping compositions according to the invention proved to be in the range of 1 to 10,000 mPa.s, preferably about 1 to about 5,000 mPa.s, measured at 20°C in a Brookfield viscosimeter (no. 5 spindle), whereas the viscosity suited for the straightening compositions is preferably higher in a range up to 50,000 mPa.s, preferably up to 30,000 mPa.s measured at 20°C in a Brookfield viscosimeter (no. 5 spindle).

The viscosity is adjusted by addition of the appropriate amounts of thickening agents known per se, such as cellulose derivatives. Thickening may as well be realized by formulating a composition in form of an emulsion with the use of C₁₀-C₂₂-fatty alcohols, in mixture with long mono alkyl chain quaternary ammonium surfactants.

Permanent shaping compositions according to the present invention preferably comprise surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, permanent shaping compositions comprise at least one cationic surfactant according to general formula 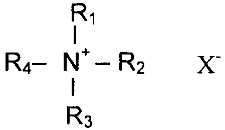
where R₁s a saturated or unsaturated, branched or non-branched alkyl chain with 8-24 C atoms or

   R₅CONH(CH₂)ₙ

   where R₅ is saturated or unsaturated, branched or non-branched alkyl chain with 7-21 C atoms and n has typical value of 0 - 4 or

   R₆COO(CH₂)ₙ

   where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-24 C atoms or

   R₅CONH(CH₂)ₙ

   where R₅ is saturated or unsaturated, branched or non-branched alkyl chain with 7-21 C atoms and n has typical value of 0 - 4 or

   R₆CO(CH₂)ₙ

   where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms which may be substituted with one or two hydroxyl group, and X is chloride, bromide or methosulfate.

Concantration of cationic surfactant is in the range from 0.05 % to 5 %, preferably 0.1 % to 2.5 % by weight, calculated to total composition.

Suitable long-chain quaternary ammonium compounds are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

Further permanent shaping compositions of the present invention may comprise additional cationic polymer. Basically suitable are all cationic polymers listed under the generic name "Polyquaternium" in the CTFA International Cosmetic Ingredient Dictionary. Examples are Polyquaternium 2, Polyquaternium 4, Polyquaternium 5, Polyquaternium 6, Polyquaternium 7, Polyquaternium 10, Polyquaternium 11, Polyquaternium 16, Polyquaternium 22 and Polyquaternium 28, and Polyquaternium 39,

The cationic polymers also include the quaternized products of graft polymers from organopolysiloxanes and polyethyl oxazolines described in EP-A 524 612 and EP-A 640 643. Such polymer is known with its CTFA name Polysilicone-9.

Concantration of one or more additional cationic polymers is in the range from 0.05 % to 2.5 %, preferably 0.1 % to 1.5 % by weight, calculated to total composition.

Permanent shaping compositions of present invention can comprise additionally at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

Permanent shaping composition of the present invention can comprise further ceramide type of compound such as cetyl-PG-hydroxyethylpalmitamide.

Further optional ingredient are sterols,especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C10 to C22 may be incorporated into the compositions of the present invention at a concentration of preferably 0.01 to 2.5% by weight calculated to total composition.

Another preferred compound in the permanent shaping composition of the present invention is silicone compounds and especially aminated silicones such as amodimethicone available from for example Dow Corning under the brand names Dow Corning 949 Emulsion and Dow Corning 2-8194. Concentration of silicones, especially amodimethicone, is in the range of 0.05 to 2.5%, preferably 0.1 to 1% by weight calculated to total composition.

Additionally, one or more natural oil component may be incorporated into the compositions of the present invention. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total composition.

Further additional compounds may be present in the permanent shaping compositions of the present invention is ubichinone of the formula 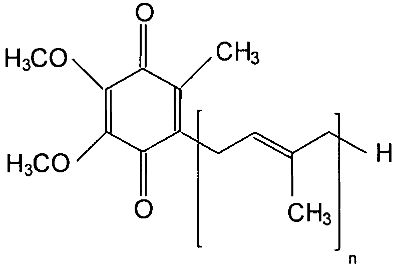 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in permanent shaping compositions of the present invention is from 0.0001 to 1%, preferably from 0.0002 to 0.75%, more preferably from 0.0002 to 0.5% and most preferably from 0.0005 to 0.5% by weight, calculated to total composition.

In another preferred embodiment of the present invention, composition comprises one or more fatty alcohol with a chain length of 12 to 24 C atoms. Concentration of one or more fatty alcohol is in the range of 1 to 15%, preferably 1 to 10%, more preferably 2 to 7.5 and most preferably 2 to 5% by weight calculated to total composition.

Suitable non limiting examples are lauryl alchohol, myristyl alcohol, cetyl alcohol, stearyl alcohol cetearyl alcohol, behenyl alcohol and their mixtures. Preferred are cetyl alcohol, stearyl alcohol cetearyl alcohol, behenyl alcohol and their mixtures. Particularly preferred are cetyl alcohol, stearyl alcohol and their 1 to 1, by weight, mixture cetearyl alcohol.

The compositions used according to the invention can naturally comprise all the substances customarily found in permanent shaping compositions, a list of which will not be given here, and are preferably present as solutions, gels with a higher or lower viscosity, emulsions or creams. They can be single-phase products or compositions packed into separate packaging which are united upon application, as they are disclosed, for example, in DE-C 43 04 828.

In order to avoid repetition, reference is here made to the state of the art as it is described, for example, in "Ullmann's Encyclopedia of Industrial Chemistry", Vol. A12 (1986), pages 588 to 591, and in particular to the monography of K. Schrader, "Grundlagen und Rezepturen der Kosmetika", 2nd. Ed. (1989, Hüthig Buchverlag) pages 823 to 840, as well as the article by D. Hollenberg et. al. in "Seifen-Öle-Fette-Wachse", 117 (1991), pages 81 to 87.

Composition of the present invention is used in a process for permanent waving wherein hair is washed or shampooed first and wound on the curlers, subsequently a reducing agent composition comprising at least one reducing agent, a cationic polymer consisting of copolymer of vinylpyrrolidone and vinylimidazole and a homopolymer of dimethyl diallyl ammonium chloride is applied onto hair and after 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers removed from hair. In cases where additional conditioning composition is required this may as well be applied after finishing the process as described above.

In another process as described above the curlers are removed after rinsing off the reducing agent and before applying the oxidizing agent.

Further in another process, after rinsing off the reducing agent from hair, an intermediate treatment composition is applied onto hair and without rinsing off but after removing the excess amount of intermediate treatment with a towel, oxidizing composition is applied and at the end of the processing they are rinsed off from hair and curlers are removed from hair.

It has further been found out that the use of cationic polymer consisting of a copolymer of vinylpyrrolidone and vinylimidazole and a homopolymer of dimethyl diallyl ammonium chloride in the intermediate treatment composition improves the permanent shaping of hair as well in terms of curl appearance and natural look and feel of hair. Therefore, in another preferred form of the present invention, permanent shaping of hair is carried our with a process wherein hair is washed or shampooed first and subsequently a reducing agent comprising composition is applied onto hair and after 1 to 45 min, preferably 1 to 30 min of processing time, depending on the hair strength, rinsed off from hair with tap water and an intermediate treatment composition comprising cationic polymer consisting of a copolymer of vinylpyrrolidone and vinylimidazole and a homopolymer of dimethyl diallyl ammonium chloride is applied and without rinsing off an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off. In cases where additional conditioning composition is required this may as well be applied after finishing the above process. In case of permanent waving hair in the above process curlers are put onto hair before application of reducing composition and taken off from hair before application of oxidizing composition or after application and processing of the oxidizing composition.

The intermediate treatment composition has a pH value between 2.5 to 6, preferably 3 to 5.5 and most preferably 3 to 5.

A straightening process may also be carried out in a different process wherein hair is washed and/or shampooed and dried and reducing composition comprising at least one reducing compound and a cationic polymer cosositing of a copolymer of vinylpyrrolidone and vinylimidazole and a homopolymer of dimethyl diallyl ammonium chloride is applied onto dry hair and processed for 5 to 60 min, preferably 5 to 45 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

The following examples are to illustrate the invention, but not to limit it.

### Example 1:

| | |
|---|---|
| Ammonium thioglycolate (60%) | 21.3 (% by wt. |
| Ammonium hydrogen carbonate | 5.0 |
| 1,3- butylene gylcol | 3.0 |
| Polyquaternium-87 | 0.5 |
| PEG-40-Hydrogenated castor oil | 0.7 |
| Perfume | 0.4 |
| Ammonia, 25% | ad pH 8.3 |
| Water | ad 100.0 |

| | |
|---|---|
| * Cationic polymer used was Luviquat Sensation from BASF. | |

With this composition the hair was permanently waved for about 15 minutes, rinsed and neutralized for about 8 minutes with a customary 2.5% H₂O₂ composition. Homogeneous wave appearance was obtained. Exclusion of Polyquaternium 87 resulted in less homogeneous perm appearance.

In all of the following examples, Luviquat Sensation from BASF was used as the cationic polymer.

### Example 2:

| | |
|---|---|
| Ammonium thioglycolate (60%) | 20 (% by wt. |
| Ammonium hydrogen carbonate | 4.0 |
| 1,3- butylene gylcol | 3.0 |
| Polyquaternium-87 | 0.25 |
| PEG-40-Hydrogenated castor oil | 0.7 |
| Perfume | 0.4 |
| Ammonia, 25% | ad pH 8.3 |
| Water | ad 100.0 |

With this composition the hair was permanently waved for about 15 minutes, rinsed and neutralized for about 8 minutes with a customary 2.5% H₂0₂ composition. Homogeneous wave appearance was obtained. Exclusion of Polyquaternium-87 resulted in less homogeneous perm appearance.

### Example 3:

### Alkaline Permanent Wave for Damaged Hair

| | |
|---|---|
| Ammonium thioglycolate (60%) | 15.0 (% by wt.) |
| Ammonium hydrogen carbonate | 2.5 |
| Ceteth-20 | 0.7 |
| Cetrimonium chloride | 0.1 |
| 1,3- butylene gylcol | 0.5 |
| Polyquaternium-87 | 0.9 |
| Perfume | 0.4 |
| Ammonia, 25% | ad pH 8.0 |
| Water | ad 100.0 |

The permanent wave achieved with this composition was similar to the one obtained with the composition according to Example 1.

Exclusion of the Polyquaternium-87 led to waves with substantially weaker contours.

### Example 4:

| | |
|---|---|
| Ammonium thioglycolate (60%) | 0.9 (% by wt.) |
| Cystein hydrochloride | 5.7 |
| Ammonium hydrogen carbonate | 1.5 |
| Acetylcystein | 0.7 |
| Cetrimonium chloride | 0.1 |
| 1,3- butylene gylcol | 0.5 |
| Polyquaternium-87 | 1.2 |
| Amodimethicone | 0.2 |
| Coenzyme Q10 | 0.05 |
| Oleic acid | 0.05 |
| Perfume | 0.4 |
| Ammonia, 25% | ad pH 9.8 |
| Water | ad 100.0 |

The permanent wave achieved with this composition was similar to the one obtained with the composition according to Example 1.

Exclusion of the Polyquaternium-87 led to substantially weaker waves.

### Example 5:

A permanent waving product consisting of two Compositions A and B, filled into a two-chamber packaging the chambers of which were kept separate until application, was prepared by destruction of the separating wall and applied onto human hair rolled onto curlers. The hair was rinsed after about fifteen minutes processing and neutralized for about five minutes with a 2.5 % H₂O₂ neutralizer composition, rinsed again, shampooed and dried.

An expressive, even, intensive permanent wave was obtained.

An identical treatment which had no Polyquaternium-87 showed a visibly inferior wave.

### Composition A:

| | |
|---|---|
| Ammonium hydrogen carbonate | 4.5 (g) |
| Polyquaternium-87 | 1.0 |
| PEG-65-Hydrogenated castor oil | 0.8 |
| Isopropyl alcohol | 1.5 |
| Ethoxydiglycol | 2.0 |
| Cocoamidopropyl betaine | 1.0 |
| Perfume | 0.3 |
| Coenzyme Q10 | 0.05 |
| Turbidifying agent | 0.5 |
| Ammonia, 25% | ad pH 8.4 |
| Water | ad 72.0 |

### Composition B:

| | |
|---|---|
| Ammonium thioglycolate, 70% | 18.0 (g) |
| Thiolactic acid | 2.0 |
| 2-Methyl-1.3-propanediol | 0.5 |
| Ammonia, 25% | ad pH 5.5 |
| Water | ad 28.0 |

After mixing of both Compositions, a ready-to-use product with a pH-value of 7.4 was obtained.

### Example 6:

A permanent waving product filled into a two-chamber package was prepared in analogy to Example 4:

### Composition A:

| | |
|---|---|
| Ammonium hydrogen carbonate | 3.5 (g) |
| Polyquaternium-87 | 0.5 |
| Ethanol | 0.5 |
| 1-Methoxypropanol | 1.0 |
| Cocoamidopropyl betaine | 1.0 |
| PEG-25-glyceryl cocoate | 0.8 |
| Coenzyme Q10 | 0.1 |
| Oleic acid | 0.05 |
| Perfume | 0.3 |
| Turbidifying agent | 0.5 |
| Ammonia, 25% | ad pH 8.3 |
| Water | ad 72.0 |

### Composition B:

| | |
|---|---|
| Ammonium thioglycolate, 70% | 13.0 (g) |
| Thiolactic acid | 0.5 |
| 2-Methyl-1.3-propanediol | 1.5 |
| Ammonia, 25% | ad pH 5.5 |
| Water | ad 28.0 |

A product with a pH-value of 7.4 was obtained by mixing of the Compositions immediately prior to application. After application onto dyed hair as described in Example 3, this mixture resulted in an expressive permanent wave, which had not effect whatever on the color gloss and color intensity.

### Example 7:

### Alkaline Permanent Waving Gel

| | |
|---|---|
| Ammonium thioglycolate, 70% | 15.0 (g) |
| Ammonium hydrogen carbonate | 4.5 |
| PEG-40-Hydrogenated castor oil | 0.7 |
| C₁₂-C₁₈-Fatty alcohol mixture | 3.5 |
| Cetrimonium chloride | 2.0 |
| Amodimethicone | 0.05 |
| 2-Methyl-1.3-propanediol | 0.5 |
| Polyquaternium-87 | 0.45 |
| Coenzyme Q10 | 0.1 |
| Perfume | 0.3 |
| Ammonia, 25% | ad pH 8.0 |
| Water | ad 100.0 |

### Intermediate treatment composition

| | |
|---|---|
| Asparagic acid | 0.25% by weight |
| Glutamic acid | 0.50 |
| Alanin DL | 0.25 |
| Magnesium sulfate | 10.00 |
| Polyquaternium-87 | 0.25 |
| Water | q.s. to 100 |

The above composition had a pH of 4.10.

### Example 8:

### Straightening Composition

| | |
|---|---|
| Thioglycolic acid | 8.0 (% by wt.) |
| C₁₆-C₂₂-Fatty alcohol mixture | 3.5 |
| Oleth-50 | 2.5 |
| Laureth-23 | 1.5 |
| Polyquaternium-87 | 0.9 |
| Ethanol | 5.0 |
| Perfume | 0.6 |
| Monoethanolamine | ad pH 9.3 |
| Water | ad 100.0 |

This composition constitutes an effecting smoothing composition for kinky hair.

## Claims

1. Composition for the permanent shaping of human hair, **characterized in that** it comprises at least one reducing agent and Polyquaternium-87as a cationic polymer.

2. Composition according to claim 1 **characterized in that** the reducing agents are selected from thioglycolic acid, thiolactic acid and their salts, cystein and its hydrochloride salt, acetylcystein.

3. Composition according to claims 1 and 2 **characterized in that** it comprises reducing agent at a concentration of 0.5 to 15% by weight and cationic polymer at a concentration of 0.1 to 2.5 % by weight, calculated to total composition.

4. Composition according to any of the preceding claims **characterized in that** cationic polymer has a cationic charge density of between 3 and 4 meq/g at pH 7.0.

5. Composition according to any of the preceding claims, **characterized in that** it has a Brookfield viscosity of 1 to 50,000 mPa.s at 20°C.

6. Composition according to any of the preceding claims **characterized in that** it comprises at least one surfactant selected from anionic, nonionic, cationic and amphoteric ones at a concentration of 0.05 to 10% by weight, calculated to total composition.

7. Composition according to any of the preceding claims **characterized in that** it comprises at least one fatty alcohol.

8. Composition according to any of the preceding claims **characterized in that** it comprises at least one organic solvent at a concentration of 0.1 to 10% by weight, calculated to total composition.

9. Composition according to any of the preceding claims **characterized in that** it comprises at least one ubichinone of the formula 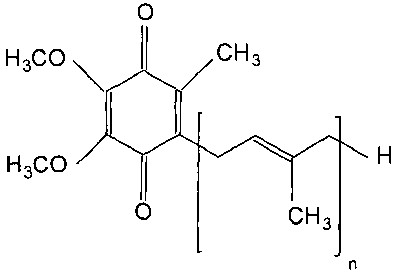 where n is a number between 1 and 10.

10. Composition according to any of the preceding claims **characterized in that** it comprises at least one silicone compound, preferably aminated silicone at a concentration of 0.05 to 2.5% by weight, calculated to total composition.

11. Composition according to any of the preceding claims **characterized in that** it has a pH in the range of 6.5 to 10.5.

12. Composition according to any of the preceding claims **characterized in that** it comprises at least one thickening agent.

13. Process for permanent waving hair **characterized in that** hair is washed or shampooed first and wound on curlers and subsequently a composition according to claims 1 to 11 is applied onto hair and at the end of the 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers are removed from hair.

14. Process according to claim 13 **characterized in that** after rinsing off the reducing agent from hair, an intermediate treatment composition optionally comprising Polyquaternium-87 is applied onto hair and without rinsing off and after removal of the excess amount with towel, an oxidizing composition is applied and at the end of the processing they are rinsed off from hair.

15. Process for straightening hair **characterized in that** hair is washed and/or shampooed and dried and reducing composition according to claims 1 to 11 is applied onto dry hair and processed for 5 to 60 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

## Patentansprüche

1. Zusammensetzung zur dauerhaften Verformung des menschlichen Haares, **dadurch gekennzeichnet, dass** sie mindestens ein Reduktionsmittel und Polyquaternium 87 als ein kationisches Polymer aufweist.

2. Zusammensetzung nach Anspruch 1, **dadurch gekennzeichnet, dass** die Reduktionsmittel aus Thioglykolsäure, Thiomilchsäure und deren Salzen, Cystein und seinem Hydrochloridsalz, Acetylcystein ausgewählt sind.

3. Zusammensetzung nach Anspruch 1 und 2, **dadurch gekennzeichnet, dass** sie Reduktionsmittel in einer Konzentration von 0,5 Gewichts-% bis 15 Gewichts-% und kationisches Polymer in einer Konzentration von 0,1 Gewichts-% bis 2,5 Gewichts-% aufweist, bezogen auf die Gesamtzusammensetzung.

4. Zusammensetzung nach einem der vorhergehenden Ansprüche, **dadurch gekennzeichnet, dass** das kationische Polymer eine kationische Ladungsdichte von 3 meq/g bis 4 meq/g bei pH 7,0 aufweist.

5. Zusammensetzung nach einem der vorhergehenden Ansprüche, **dadurch gekennzeichnet, dass** sie eine Brookfield-Viskosität von 1 mPa·s bis 50.000 mPa·s bei 20 °C aufweist.

6. Zusammensetzung nach einem der vorhergehenden Ansprüche, **dadurch gekennzeichnet, dass** sie mindestens ein Tensid, ausgewählt aus anionischen, nichtionischen, kationischen und amphoteren, in einer Konzentration von 0,05 Gewichts-% bis 10 Gewichts-% aufweist, bezogen auf die Gesamtzusammensetzung.

7. Zusammensetzung nach einem der vorhergehenden Ansprüche, **dadurch gekennzeichnet, dass** sie mindestens einen Fettalkohol aufweist.

8. Zusammensetzung nach einem der vorhergehenden Ansprüche, **dadurch gekennzeichnet, dass** sie mindestens ein organisches Lösungsmittel in einer Konzentration von 0,1 Gewichts-% bis 10 Gewichts-% aufweist, bezogen auf die Gesamtzusammensetzung.

9. Zusammensetzung nach einem der vorhergehenden Ansprüche, **dadurch gekennzeichnet, dass** sie mindestens ein Ubichinon der Formel 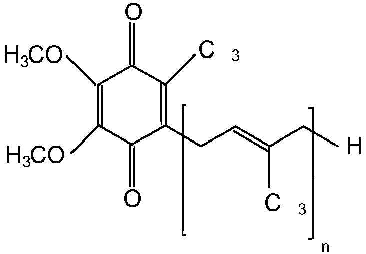 aufweist, wobei n eine Zahl von 1 bis 10 ist.

10. Zusammensetzung nach einem der vorhergehenden Ansprüche, **dadurch gekennzeichnet, dass** sie mindestens eine Silikonverbindung aufweist, vorzugsweise aminiertes Silikon in einer Konzentration von 0,05 Gewichts-% bis 2,5 Gewichts-%, bezogen auf die Gesamtzusammensetzung.

11. Zusammensetzung nach einem der vorhergehenden Ansprüche, **dadurch gekennzeichnet, dass** sie einen pH-Wert im Bereich von 6,5 bis 10,5 aufweist.

12. Zusammensetzung nach einem der vorhergehenden Ansprüche, **dadurch gekennzeichnet, dass** sie mindestens ein Verdickungsmittel aufweist.

13. Verfahren zum dauerhaften Wellen des Haares, **dadurch gekennzeichnet, dass** das Haar zunächst gewaschen oder shampooniert und auf Lockenwickler gezogen wird und anschließend eine Zusammensetzung nach Anspruch 1 bis 11 auf das Haar aufgebracht wird und am Ende der 1- bis 45-minütigen, vorzugsweise 1- bis 30-minütigen, Einwirkzeit, abhängig von der Stärke des Haares, mit Leitungswasser aus dem Haar ausgespült wird und eine oxidierende Zusammensetzung, welche mindestens ein Oxidationsmittel aufweist, vorzugsweise Wasserstoffperoxid oder Natriumbromat in einer Konzentration von 0,5 Gewichts-% bis 10 Gewichts-%, bezogen auf die gesamte oxidierende Zusammensetzung, auf das Haar aufgebracht und für 1 Minute bis 20 Minuten auf dem Haar belassen und ausgespült wird und die Lockenwickler aus dem Haar entfernt werden.

14. Verfahren nach Anspruch 13, **dadurch gekennzeichnet, dass** nach dem Ausspülen des Reduktionsmittels aus dem Haar eine Zwischenbehandlungszusammensetzung, welche gegebenenfalls Polyquaternium 87 aufweist, auf das Haar aufgebracht wird und ohne Ausspülen und nach dem Entfernen der überschüssigen Menge mit einem Handtuch eine oxidierende Zusammensetzung aufgebracht wird und sie am Ende der Einwirkung aus dem Haar ausgespült werden.

15. Verfahren zum Glätten des Haares, **dadurch gekennzeichnet, dass** das Haar gewaschen und/oder shampooniert und getrocknet wird und eine reduzierende Zusammensetzung nach Anspruch 1 bis 11 auf das trockene Haar aufgebracht wird und man sie 5 Minuten bis 60 Minuten lang einwirken lässt und sie mit Wasser ausgespült und getrocknet wird und das trockene Haar physikalisch mit einem Glätteisen bei einer Temperatur von 130 °C bis 210 °C geglättet wird und anschließend eine oxidierende Zusammensetzung, welche mindestens ein Oxidationsmittel aufweist, vorzugsweise Wasserstoffperoxid oder Natriumbromat in einer Konzentration von 0,5 Gewichts-% bis 10 Gewichts-%, bezogen auf die gesamte oxidierende Zusammensetzung, auf das Haar aufgebracht und 1 Minute bis 20 Minuten lang auf dem Haar belassen und ausgespült wird.

## Revendications

1. Composition de mise en forme permanente de cheveux humains, **caractérisée en ce qu'**elle comprend au moins un agent réducteur et du Polyquaternium-87 en tant que polymère cationique.

2. Composition selon la revendication 1, **caractérisée en ce que** les agents réducteurs sont choisis parmi l'acide thioglycolique, l'acide thiolactique et leurs sels, la cystéine et son sel chlorhydrate, l'acétylcystéine.

3. Composition selon les revendications 1 et 2, **caractérisée en ce qu'**elle comprend l'agent réducteur à une concentration en poids de 0,5 à 15 % et le polymère cationique à une concentration en poids de 0,1 à 2,5 %, calculées par rapport à la composition totale.

4. Composition selon l'une quelconque des revendications précédentes, **caractérisée en ce que** le polymère cationique a une densité de charge cationique entre 3 et 4 meq / g à pH 7,0.

5. Composition selon l'une quelconque des revendications précédentes, **caractérisée en ce qu'**elle a une viscosité Brookfield de 1 à 50 000 mPa·s à 20 °C.

6. Composition selon l'une quelconque des revendications précédentes, **caractérisée en ce qu'**elle comprend au moins un tensioactif choisi parmi les tensioactifs anioniques, non ioniques, cationiques et amphotères, à une concentration en poids de 0,05 à 10 %, calculée par rapport à la composition totale.

7. Composition selon l'une quelconque des revendications précédentes, **caractérisée en ce qu'**elle comprend au moins un alcool gras.

8. Composition selon l'une quelconque des revendications précédentes, **caractérisée en ce qu'**elle comprend au moins un solvant organique à une concentration en poids de 0,1 à 10 %, calculée par rapport à la composition totale.

9. Composition selon l'une quelconque des revendications précédentes, **caractérisée en ce qu'**elle comprend au moins une ubiquinone selon la formule 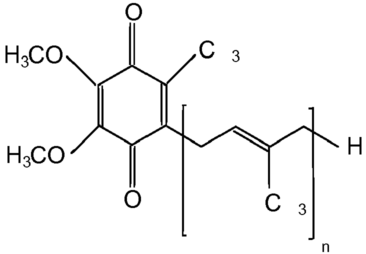 où n est un nombre entre 1 et 10.

10. Composition selon l'une quelconque des revendications précédentes, **caractérisée en ce qu'**elle comprend au moins un composé silicone, de préférence un silicone aminé à une concentration en poids de 0,05 à 2,5 %, calculée par rapport à la composition totale.

11. Composition selon l'une quelconque des revendications précédentes, **caractérisée en ce qu'**elle a un pH dans la plage de 6,5 à 10,5.

12. Composition selon l'une quelconque des revendications précédentes, **caractérisée en ce qu'**elle comprend au moins un agent épaississant.

13. Procédé de mise en plis permanente de cheveux **caractérisé en ce que** les cheveux sont d'abord lavés ou shampouinés et enroulés sur des bigoudis, et ensuite une composition selon les revendications 1 à 11 est appliquée sur les cheveux et, à la fin d'un temps de traitement de 1 à 45 minutes, de préférence de 1 à 30 minutes, en fonction de la vitalité des cheveux, est éliminée des cheveux par rinçage à l'eau du robinet et une composition oxydante comprenant au moins un agent oxydant, de préférence du peroxyde d'hydrogène ou du bromate de sodium, à une concentration en poids de 0,5 à 10 % calculée par rapport à la totalité de la composition oxydante, est appliquée sur les cheveux et laissée sur les cheveux de 1 à 20 minutes, et est éliminée par rinçage, et les bigoudis sont retirés des cheveux.

14. Procédé selon la revendication 13, **caractérisé en ce qu'**à l'issue de l'élimination de l'agent réducteur par le rinçage des cheveux, une composition de traitement intermédiaire comprenant éventuellement du Polyquaternium-87 est appliquée sur les cheveux et, sans rinçage et après élimination de la quantité en excès avec une serviette, une composition oxydante est appliquée, et, à la fin du traitement, elles sont éliminées par rinçage des cheveux.

15. Procédé pour défriser des cheveux **caractérisé en ce que** les cheveux sont lavés et/ou shampouinés et séchés, et la composition réductrice selon les revendications 1 à 11 est appliquée sur les cheveux secs et pour une durée de traitement de 5 à 60 minutes, et les cheveux sont rincés à l'eau et séchés, et les cheveux secs sont défrisés physiquement au fer chaud à une température de 130 à 210 °C, et ensuite une composition oxydante comprenant au moins un agent oxydant, de préférence du peroxyde d'hydrogène ou du bromate de sodium, à une concentration en poids de 0,5 à 10 %, calculée par rapport à la totalité de la composition oxydante, est appliquée sur les cheveux et laissée sur les cheveux pendant 1 à 20 minutes, et est éliminée par rinçage.
